Europäisches Patentamt

European Patent Office

Office européen des brevets

(11) Veröffentlichungsnummer: **0 510 593 A1**

## (12) EUROPÄISCHE PATENTANMELDUNG

(21) Anmeldenummer: **92106842.5**

(51) Int. Cl.⁵: **A61F 13/10**

(22) Anmeldetag: **22.04.92**

(30) Priorität: **26.04.91 DE 9105369 U**

(43) Veröffentlichungstag der Anmeldung:
**28.10.92 Patentblatt 92/44**

(84) Benannte Vertragsstaaten:
**AT CH DE FR GB IT LI**

(71) Anmelder: **Kleylein, Horst**
**Glockendonstrasse 6**
**W-8500 Nürnberg 80(DE)**
Anmelder: **Kleylein, Harald**
**Glockendonstrasse 6**
**W-8500 Nürnberg 80(DE)**

(72) Erfinder: **Kleylein, Horst**
**Glockendonstrasse 6**
**W-8500 Nürnberg 80(DE)**
Erfinder: **Kleylein, Harald**
**Glockendonstrasse 6**
**W-8500 Nürnberg 80(DE)**

(74) Vertreter: **Merten, Fritz**
**Tristanstrasse 5**
**W-8500 Nürnberg 40(DE)**

(54) **Ellenbogenorthese.**

(57) Eine Ellenbogenorthese aus einer formgestrickten Zweizugbandage weist beidseitig unterhalb ihres Beugebereiches (2) viskoelastische Polstereinlagen (6) auf. Beide Polstereinlagen (6) sind räumlich symmetrische Gebilde mit etwa der Ellenbogengelenkebene (7) als Symmetrieebene.

FIG.2

Die Erfindung betrifft eine Ellenbogenorthese mit den im Oberbegriff des Anspruches 1 genannten Merkmalen.

Bei einer bekannten Orthese der eingangs genannten Art sind die beiden viskoelastischen Profileinlagen unterschiedlich ausgestaltet. Dabei ist die die radialen Muskelgruppen des Unterarmes beaufschlagende Polstereinlage als Profileinlage um den radialen Epicondylus herumgelegt und erstreckt sich dazu bis in den Beugebereich der Bandage. Das Anziehen bzw. Überstreifen dieser bekannten Bandage erfordert besondere Sorgfalt vor allen Dingen bei der Positionierung der Profileinlage im Bereich um den radialen Epicondylus. Darüber hinaus haben neuere Erkenntnisse die Umfassung des radialen Epicondylus durch die viskoelastische Profileinlage als therapeutisch wenig wirksam erwiesen. Ein besonderer Nachteil dieser bekannten Bandage besteht indessen darin, daß sie nicht in gleicher Weise für den linken wie für den rechten Arm anwendbar ist.

Die Erfindung schlägt demgegenüber eine Ellenbogenorthese der eingangs genannten Art mit zusätzlich den Merkmalen des Kennzeichens des Patentanspruches 1 vor. Neben verbesserter therapeutischer Wirkung besteht ein Vorteil darin, daß dieselbe Orthese sowohl für den linken wie für den rechten Arm in gleicher Weise anwendbar ist. Dies begünstigt eine Massenfertigung und erleichtert auch die Lagerhaltung im Handel, ganz abgesehen von den damit für den Endabnehmer verbundenen Gebrauchsvorteilen.

Weitere Erfindungsmerkmale werden anhand eines in den Figuren dargestellten Ausführungsbeispiels näher beschrieben. Es zeigen:

Fig. 1)   eine Seitenansicht der Orthese in nicht angelegter Ruhelage;

Fig. 2)   eine Frontansicht in Pfeilrichtung II von Fig. 1);

Fig. 3)   einen vergrößerten Schnitt entsprechend der Linie III-III in Fig. 1);

Fig. 4)   einen Schnitt entsprechend Fig. 3) durch die allerdings in Transport- und Lagerstellung zusammengelegte Ellenbogenorthese;

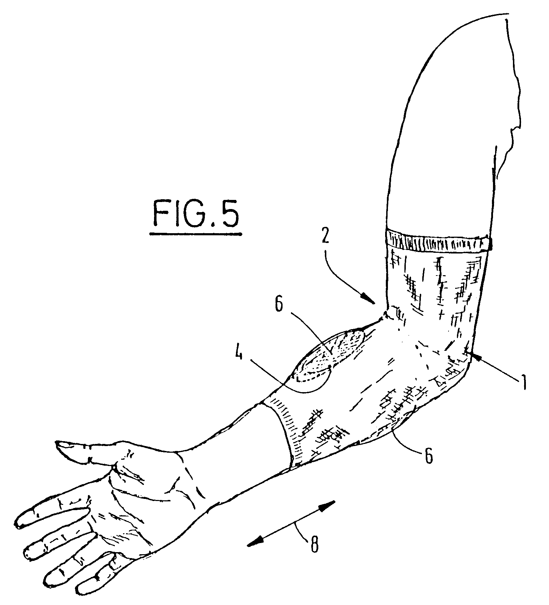

Fig. 5)   die perspektivische Darstellung des Armes einer Trageperson mit angelegter Ellenbogenorthese.

Die Ellenbogenorthese besteht aus einer anatomisch in funktioneller Armbeugestellung formgestrickten, schlauchartigen Zweizugbandage 1 mit beidseitig unterhalb ihres Beugebereiches 2 über der radialen und ulnaren Muskulatur angeordneten, schichtartig auf der Bandageninnenoberfläche 3 aufliegenden und in Auflagestellung durch eine mit den Nähten 4 aufgenähte Textilstoffschicht 5 in Auflagestellung fixierten, viskoelastischen Polstereinlagen 6 zur Erzeugung eines intermittierenden Kompressionsdruckes, der gezielt auf die radialen und ulnaren Muskelgruppen wirkt. Diese eingearbeiteten Profileinlagen 6 üben unter dem Kompressionsdruck eine rhythmische Massagewirkung aus und lenken die in den Epicondylus einstrahlenden Sehnenfasern um. Die Polstereinlagen 6 bestehen aus Silikon. Die ständig massierende Wirkung der Silikoneinlage verbessert die Durchblutung und führt zur raschen Resorption von Ödemen und Hämatomen. Diese schmerzlose Dauermassage bewirkt bereits nach kurzer Tragezeit eine rasche Abschwellung und Schmerzlinderung.

Die beiden Polstereinlagen 6 sind nach Eigengestalt und Anbringung an der Bandage 1 räumlich symmetrische Gebilde mit etwa der Ellenbogengelenkebene 7 als Symmetrieebene.

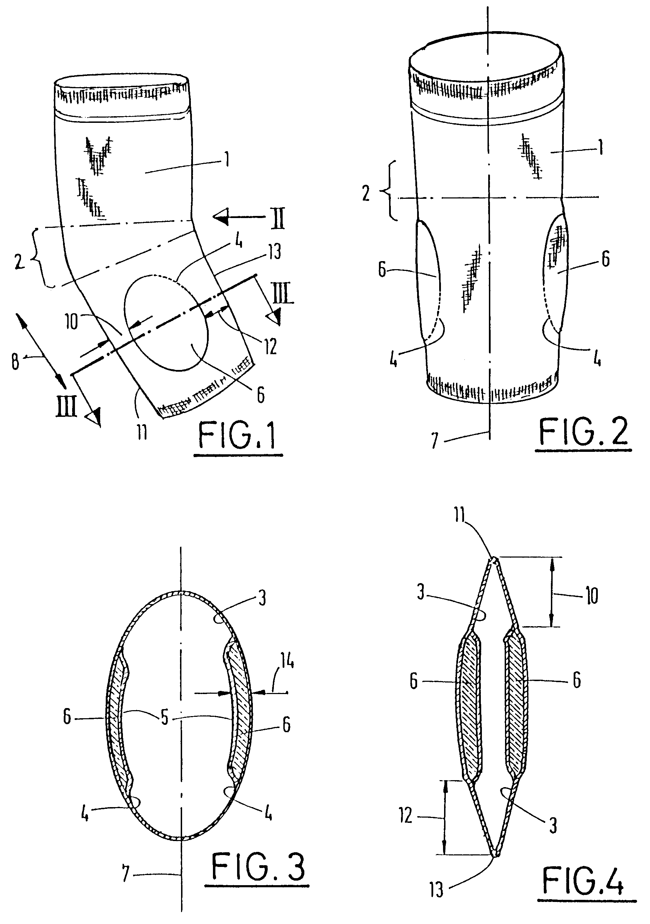

Die Polstereinlagen 6 weisen eine oval-rechteckige Umrißform auf, deren größere Längserstreckung etwa in Unterarmlängsrichtung 8 verläuft. Beide Polstereinlagen 6 insgesamt nehmen mindestens die Hälfte des Bandagenumfanges im Unterarmbereich ein. Die Umfangsrichtung des Bandagenumfanges ist durch Pfeil 9 in Fig. 3) gekennzeichnet.

Der Abstand 10 der Polstereinlagen 6 von der Bandagenaußenkante 11 ist kleiner als der entsprechende Abstand 12 von der Bandageninnenkante 13. Die Schichtdicke 14 der Polstereinlagen 6 ist gleichmäßig. Die Innenoberflächen der Polstereinlagen sind durch die Textilstoffschichten 5 abgedeckt.

**Patentansprüche**

1.   Ellenbogenorthese aus einer anatomisch in funktioneller, mittlerer Armbeugestellung formgestrickten, schlauchartigen Zweizugbandage und mit beidseitig unterhalb ihres Beugebereiches (2) über der radialen und ulnaren Muskulatur angeordneten, schichtartig auf der Bandageninnenoberfläche (3) aufliegenden und in Auflagestellung fixierten, viskoelastischen Polstereinlagen (6) zur Erzeugung eines intermittierenden Kompressionsdruckes,
**dadurch gekennzeichnet,**
daß die beiden Polstereinlagen (6) räumlich symmetrische Gebilde mit etwa der Ellenbogengelenkebene (7) als Symmetrieebene sind.

2.   Ellenbogenorthese nach Anspruch 1,
**gekennzeichnet durch**
eine oval-rechteckige Umrißform der Polstereinlagen (6), deren größere Längserstreckung etwa in Unterarmlängsrichtung (8) verläuft.

3.   Ellenbogenorthese nach Anspruch 1 oder 2,
**dadurch gekennzeichnet,**
daß beide Polstereinlagen (6) insgesamt mindestens die Hälfte des Bandagenumfanges im Unterarmbereich einnehmen.

4. Ellenbogenorthese nach einem oder mehreren der vorhergehenden Ansprüche,
**dadurch gekennzeichnet**,
daß der Abstand (10) der Polstereinlagen (6) von der Bandagenaußenkante (11) kleiner ist als von der Bandageninnenkante.

5. Ellenbogenorthese nach einem oder mehreren der vorhergehenden Ansprüche,
**gekennzeichnet durch**
eine gleichmäßige Schichtdicke (14) der Polstereinlagen (6).

6. Ellenbogenorthese nach einem oder mehreren der vorhergehenden Ansprüche,
**dadurch gekennzeichnet**,
daß die Innenoberflächen der Polstereinlagen (6) durch eine Textilstoffschicht (5) abgedeckt sind.

7. Ellenbogenorthese nach einem oder mehreren der vorhergehenden Ansprüche,
**dadurch gekennzeichnet**,
daß die viskoelastische Polstereinlage aus Silikon besteht.

FIG.1

FIG.2

FIG.3

FIG.4

FIG.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| Y | WO-A-8 604 811 (WEIHERMÜLLER & VOIGTMANN)<br>* Seite 4, Zeile 11 - Zeile 15 *<br>* Zusammenfassung *<br>* Abbildung *<br>--- | 1,2,4-6 | A61F13/10 |
| Y | FR-A-2 636 229 (ETABLISSEMENTS PICHON FRERES)<br>* Anspruch 1; Abbildungen *<br>--- | 1,2,4-6 | |
| A | DE-A-3 508 566 (STEINBERGER)<br>* Abbildung *<br>--- | 3 | |
| A | DE-A-3 902 434 (SPORTARTIKELFABRIK KARL UHL)<br>* Zusammenfassung *<br>* Abbildungen *<br>----- | 7 | |

|  |  |
|---|---|
| | **RECHERCHIERTE SACHGEBIETE (Int. Cl.5 )**<br><br>A61F |

Der vorliegende Recherchenbericht wurde für alle Patentansprüche erstellt

| Recherchenort | Abschlußdatum der Recherche | Prüfer |
|---|---|---|
| DEN HAAG | 19 JUNI 1992 | GODOT T. |

EPO FORM 1503 03.82 (P0403)